(19) Europäisches Patentamt
European Patent Office
Office européen des brevets

(11) Veröffentlichungsnummer: **0 154 225 B1**

(12) **EUROPÄISCHE PATENTSCHRIFT**

(45) Veröffentlichungstag der Patentschrift: **03.04.91**

(51) Int. Cl.⁵: **C07D 495/04**, C07D 519/00, //(C07D495/04,333:00,235:00), (C07D519/00,495:00,493:00)

(21) Anmeldenummer: **85101612.1**

(22) Anmeldetag: **14.02.85**

# (54) Verfahren zur Herstellung eines optisch aktiven Lactons und neue Zwischenprodukte in diesem Verfahren.

(30) Priorität: **09.03.84 CH 1171/84**

(43) Veröffentlichungstag der Anmeldung:
**11.09.85 Patentblatt 85/37**

(45) Bekanntmachung des Hinweises auf die Patenterteilung:
**03.04.91 Patentblatt 91/14**

(84) Benannte Vertragsstaaten:
**AT BE CH DE FR GB IT LI NL**

(56) Entgegenhaltungen:
**GB-A- 1 320 799**

(73) Patentinhaber: **F. HOFFMANN-LA ROCHE AG**
**Postfach 3255**
**CH-4002 Basel(CH)**

(72) Erfinder: **Holick, Wolfgang, Dr.**
**Schlossgasse 55**
**W-7879 Grenzach-Wyhlen(DE)**
Erfinder: **Pauling, Horst, Dr.**
**Ruchholzstrasse 39**
**CH-4103 Bottmingen(CH)**

(74) Vertreter: **Cottong, Norbert A. et al**
**Grenzacherstrasse 124 Postfach 3255**
**CH-4002 Basel(CH)**

**EP 0 154 225 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues Verfahren zur Herstellung von Verbindungen, welche als Zwischenprodukte für die Herstellung von D-(+)-Biotin geeignet sind, sowie ein Verfahren zur Herstellung von D-(+)-Biotin selbst. Die Erfindung betrifft ferner neue Zwischenprodukte in diesem Verfahren.

D-(+)-Biotin ist eine seit langem bekannte Substanz, und es sind demgemäss auch bereits eine Reihe Verfahren zu deren Herstellung bekannt. Den technisch interessanten Verfahren ist die Notwendigkeit gemeinsam, auf irgendeiner Stufe die Carboxybutylseitenkette an das Ringsystem anzuhängen. Hierfür sind verschiedene Lösungen bekannt, wie etwa der Aufbau der Seitenkette nach dem Verknüpfungsschema $C_4 + C_1 \rightarrow C_5$ S.G.B.-Patent Schrift 1320 799 oder auch $C_3 + (C_3\text{-}C_1 = C_2) \rightarrow C_5$.

Es ist auch bekannt, die Seitenkette in einer Stufe mittels einer Wittig-Reaktion an das Ringsystem anzuhängen. Alle diese Verfahren haben jedoch den Nachteil, dass sie entweder über eine verhältnismässig grosse Anzahl von Reaktionsstufen verlaufen oder aber einen relativ grossen Auf wand zur Isolierung des gewünschten Endproduktes erfordern.

Es bestand somit ein Bedürfnis nach einem technisch einfachen Verfahren, gemäss welchem die Seitenkette in guter Ausbeute und in möglichst einem Reaktionsschritt an das Ringsystem angehängt werden kann. Dies ist nunmehr mittels des erfindungsgemässen Verfahrens möglich.

Dieses Verfahren ist dadurch gekennzeichnet, dass man das Thiolacton der Formel

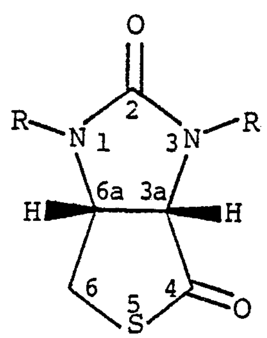

I

worin R die Benzylgruppe darstellt, mit einer Grignard-Verbindung der Formel

$$X\text{-}Mg\text{-}CH_2\text{-}CH_2\text{-}CH_2\text{-}CH_2\text{-}R^1 \qquad II$$

worin X Halogen darstellt und $R^1$ den Rest der Formel

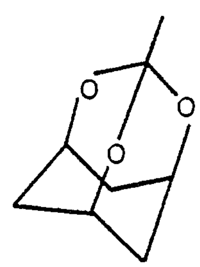

bedeutet,

umsetzt, dass man, die so erhaltene Verbindung der Formel

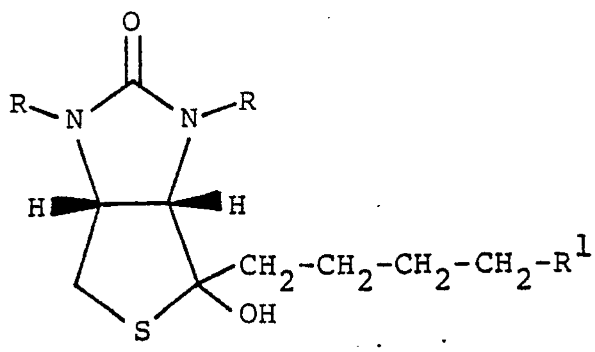

III

worin R und $R^1$ die obige Bedeutung haben, dehydratisiert, dass man, in der so erhaltenen Verbindung der Formel

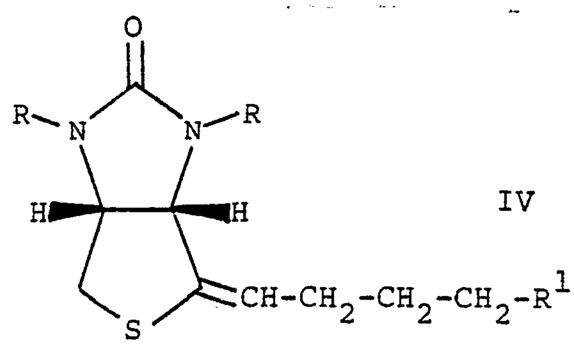

IV

worin R und $R^1$ die obige Bedeutung haben, durch Abspaltung von cis-1,3,5-Cyclohexantriol, die Carboxylgruppe in der Seitenkette freisetzt, und dass man, die so erhaltene Verbindung der Formel

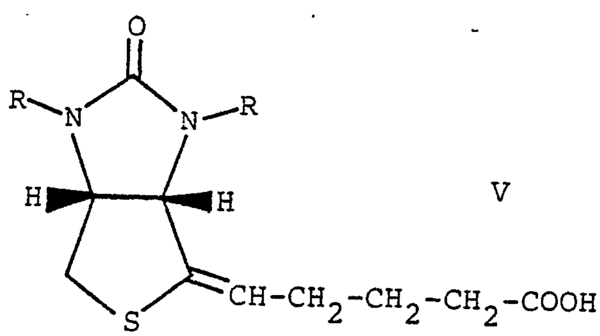

V

worin R die obige Bedeutung hat, in D-(+)-Biotin überführt.

Der Ausdruck "Halogen", bedeutet im Rahmen der vorliegenden Erfindung Chlor, Brom und Jod, wobei Brom bevorzugt ist.

Die als Ausgangsmaterialien verwendeten Verbindungen der Formeln I und II, sowie die erfindungsgemäss hergestellte Verbindung der Formel V sind bekannte Verbindungen. Die Verbindungen der Formeln III und IV sind jedoch neu und ebenfalls Gegenstand der vorliegenden Erfindung.

Die Umsetzung des Thiolactons der Formel I mit einer Grignard-Verbindung der Formel II kann in an sich bekannter weise, d.h. unter den finr eine Grignard-Reaktion üblichen Bedingungen durchgeführt werden. Zweckmässig erfolgt dies in einem unter den Reaktionsbedingungen inerten organischen Lösungsmittel, beispielsweise in einem niederen Alkyläther wie Diäthyläther oder einem cyclischen Aether wie Tetrahydrofuran, Dioxan und dergleichen und bei einer Temperatur von etwa -20° C bis zum Siedepunkt des verwendeten Lösungsmittels, vorzugsweise bei etwa 0° C bis etwa 50° C, insbesondere bei Raumtemperatur.

Die Dehydratisierung der Verbindung der Formel III kann in an sich bekannter Weise durchgefllhrt

werden. Zweckmässig erfolgt dies durch Behandlung mit einer Säure wie etwa Schwefelsäure, Salzsäure, p-Toluolsulfonsäure und dergleichen. Als Lösungsmittel wird zweckmässig ein solches verwendet, welches mit dem gebildeten Wasser ein Azeotrop bildet, z.B. aromatische Kohlenwasserstoffe wie Benzol, Toluol, Xylol und dergleichen. Die Dehydratisierung erfolgt auch vorteilhaft bei erhöhter Temperatur, vorzugsweise bei Rückflusstemperatur des Reaktionsgemisches.

Die Abspaltung des cis-1,3,5-Cyclohexantriols aus der Verbindung der Formel IV, d.h. die Freisetzung der Carboxylgruppe am $C_4$-Atom der Seitenkette, kann in an sich bekannter Weise durchgeführt werden. Zweckmässig kann dies durch Behandlung mit einer wässrigen Mineralsäure wie Schwefelsäure, Bromwasserstoffsäure, Chlorwasserstoffsäure und dergleichen erfolgen, wobei die Zugabe von katalytischen Mengen an p-Toluolsulfonsäure diese Reaktion beschleunigt. Zur Vervollständigung der Reaktion wird anschliessend das Reaktionsgemisch noch mit wässriger Alkalihydroxidlösung unter Rückfluss erhitzt. Als Alkalihydroxide können hier genannt werden Lithium-, Kalium- und Natriumhydroxyd.

Die Dehydratisierung der Verbindung der Formel III zur Verbindung der Formel IV und die anschliessende Abspaltung des cis-1,3,5-Cyclohexantriols können sowohl in einem Eintopfverfahren durchgeführt werden als auch in zwei separaten Schritten, mit dazwischenliegender Isolierung der Verbindung der Formel IV.

Die nach der Abspaltung des cis-1,3,5-Cyclohexantriols erhaltene Verbindung der Formel V ist, wie bereits erwähnt. eine bekannte Verbindung und kann in bekannter Weise, d.h. durch Hydrierung der Doppelbindung und Abspaltung der Schutzgruppen an den Stickstoffatomen, leicht in D-(+)-Biotin übergeführt werden.

Die nachfolgenden Beispiele erläutern die Erfindung.

Beispiel 1

Zu einer Lösung von 1,52 g (4.5 mMol) (+)-cis -1,3,-Dibenzyl-hexahydro-1H-thieno[3,4-d]imidazol -2,4-dion in 25 ml Tetrahydrofuran werden langsam 1,35 g (4,5 mMol) 4-(2,4,10-Trioxäadamantyl)-butylmagnesiumbromid in 45 ml Tetrahydrofuran unter Argon bei Raumtemperatur zugetropft. Das Gemisch wird noch 15 Stunden bei Raumtemperatur gerührt, dann mit 300 ml Essigester verdünnt und mit 100 ml 1N Salzsäure versetzt. Die organische Phase wird abgetrennt und mit 10 Gew.%iger Bicarbonatlösung, mit Wasser und mit gesättigter Kochsalzlösung gewaschen und über Natriumsulfat getrocknet. Der nach dem Abziehen des Lösungsmittels verbleibende Rückstand wird an Kieselgel chromatographiert. Elution mit Toluol, Toluol/Essigester (2:1) und Toluol/Essigester (1:2) ergibt zunächst 0,5 g einer weissen, wachsartigen Masse. Anschliessend werden 1,75 g (73% der Theorie) cis-1,3-Dibenzyl-4-hydroxy 4-(4-(2,4,10-trioxaadamantyl)-butyl)-hexahydro -1H-thieno[3,4-d]imidazol-2-on in Form eines weissen Pulvers eluiert. Schmelzpunkt 186-192 °C.

Beispiel 2

1,61 g (3 mMol) cis-1,3-Dibenzyl-4-hydroxy -4-(4 -(2,4,10-trioxaadamantyl)-butyl)-hexahydro -1H-thieno-[3,4-d]imidazol-2-on (hergestellt gemäss Beispiel 1) werden in 50 ml Toluol gelöst. mit 5 mg p-Toluolsulfonsäure versetzt und zum Sieden erhitzt. Innerhalb von 30 Minuten werden 20 ml Toluol abdestilliert. Nach dieser Zeit ist im Dünnschichtchromatogramm kein Ausgangsmaterial mehr nachweisbar. Die Lösung wird nun zur Trockene eingedampft. Der Rückstand wird mit 8,5 ml Dioxan aufgenommen, mit 8,5 ml 0,02N Schwefelsäure versetzt und 2,5 Stunden unter Rückfluss gekocht. Danach wird mit 2,85 ml 2N Kalilauge alkalisch gestellt und weitere 30 Minuten unter Rückfluss gekocht. Anschliessend wird auf Raumtemperatur abgekühlt und mit verdünnter Salzsäure angesäuert. Die Lösung wird sodann mit insgesamt 200 ml Essigester extrahiert. Die vereinigten Extrakte werden mit Wasser und gesättigter Kochsalzlösung gewaschen und über Natriumsulfat getrocknet. Der nach dem Abziehen des Lösungsmittels verbleibende Rückstand wird an Kieselgel chromatographiert. Elution mit Toluol, Toluol/Essigester (9:1), (8:2), (1:1), und mit reinem Essigester ergibt 955 mg (76% der Theorie) cis-2-Oxo-1,3-dibenzyl-hexahydro -1H-thieno[3,4-d]-imidazol-4-ylidenpentansäure als hellbraunes, beim Trocknen erstarrendes Oel.

$^1$H-NMR (80 MHz, $CDCl_3$): 1,4-1,9 ppm (m) und 1,9-2,5 ppm (m), 6 H, 2,96 ppm (d) 2 H, 3,8-4.4 ppm (m) 4 H, 4,82 ppm (d) und 4,95 ppm (d) 2 H, 5,43 ppm (t) 1 H, 7,3 ppm (m) 10 H, 8,5 ppm (broad) 1H. IR: 703, 754, 1181, 1234, 1495, 1583, 1657, 1696. 1732 $cm^{-1}$. MS: M = 422.

Beispiel 3

910 mg (1,68 mMol) cis-1.3-Dibenzyl-4-hydroxy-4-(4 -(2,4,10-trioxaadamantyl) butyl)-hexahydro-1H-thieno[3.4-d]imidazol-2-on (hergestellt gemäss Beispiel 1) werden in 50 ml Toluol gelöst und mit 20 mg p-Toluolsulfonsäure versetzt. Die Lösung wird zum Sieden erhitzt, und innerhalb einer Stunde werden 20 ml Toluol abdestilliert. Danach wird die Lösung zur Trockene eingedampft. Der verbleibende Rückstand wird an Kieselgel chromatographiert. Elution mit Toluol, Toluol/Essigester (9:1) und (8:2) ergibt 839 mg (96% d.Th.) cis-1,3-Dibenzyl-4-(4-(2,4,10-trioxaadamantyl) -butyliden)-hexahydro-1H-thieno[3,4-d]imidazol-2-on in Form eines leicht braunen Oeles, welches beim Trocknen erstarrt.

$^1$H-NMR (80 HHz, $CDCl_3$): 1,2-2,3 ppm (m) und 2,3-2,8 ppm (m) 12 H, 2,95 ppm (d) 2 H, 3,8-4,5 ppm (m) 7 H, 4,79 ppm (d) und 4,96 ppm (d) 2 H, 5,45 ppm (t) 1 H, 7,38 ppm (m) 10 H.

Beispiel 4

839 mg (1,62 mMol) cis-1,3-Dibenzyl-4(4-(2,4,10-trioxa-adamantyl)-butyliden)-hexahydro-1H-thieno[3,4-d]imidazol-2-on (hergestellt gemäss Beispiel 3) werden mit 4,6 ml Dioxan, 4,6 ml 0,02N Schwefelsäure sowie 20 mg p-Toluolsulfonsäure versetzt und 2,5 Stunden unter einer Argonatmosphäre zum Sieden erhitzt. Anschliessend wird mit 1,54 ml 2N Kalilauge alkalisch gestellt und weitere 45 Minuten unter Rückfluss gekocht. Nach dem Abkühlen auf Raumtemperatur wird die Lösung mit Essigester extrahiert und der Extrakt verworfen. Die wässrige Phase wird mit verdünnter Salzsäure angesäuert und erneut mit insgesamt 200 ml Essigester extrahiert. Die vereinigten Extrakte werden einmal mit gesättigter Kochsalzlösung gewaschen und über Natriumsulfat getrocknet. Nach dem Abziehen des Lösungsmittels erhält man 622 mg (89% der Theorie) cis-2-Oxo-1,3-dibenzyl-hexahydro-1H-thieno[3,4-d]imidazol-4-ylidenpentansäure.

## Ansprüche

**1.** Verfahren zur Herstellung von D-(+)-Biotin, dadurch gekennzeichnet, dass man das Thiolacton der Formel

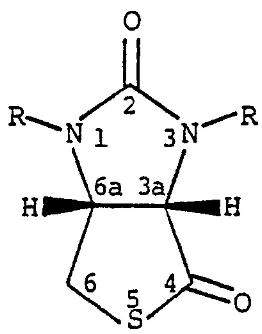

I

worin R die Benzylgruppe darstellt, mit einer Grignard-Verbindung der Formel

$$X\text{-}Mg\text{-}CH_2\text{-}CH_2\text{-}CH_2\text{-}CH_2\text{-}R^1 \qquad II$$

worin X Halogen darstellt und $R^1$ den Rest der Formel

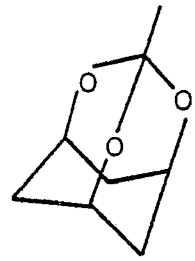

bedeutet,
umsetzt, dass man, die so erhaltene Verbindung der Formel

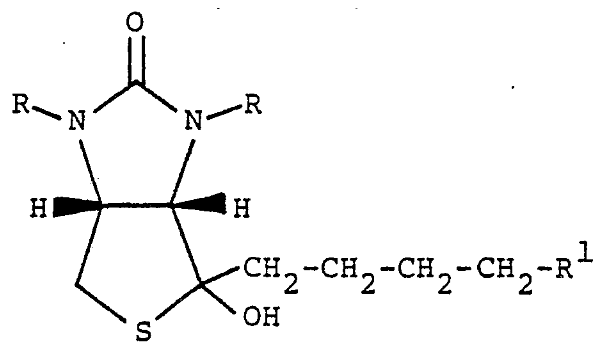

III

worin R und $R^1$ die obige Bedeutung haben, dehydratisiert, dass man, in der so erhaltenen Verbindung der Formel

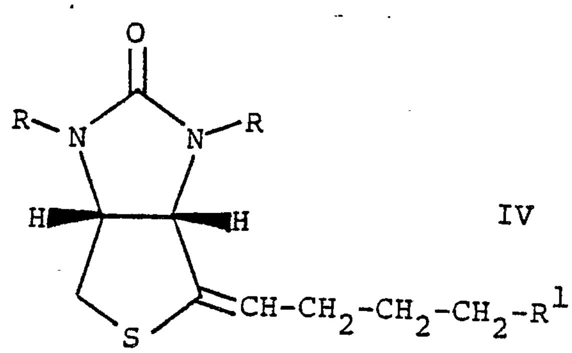

IV

worin R und $R^1$ die obige Bedeutung haben, durch Abspaltung von cis-1,3,5-Cyclohexantriol, die Carboxylgruppe der Seitenkette freisetzt, und die so erhaltene Verbindung der Formel

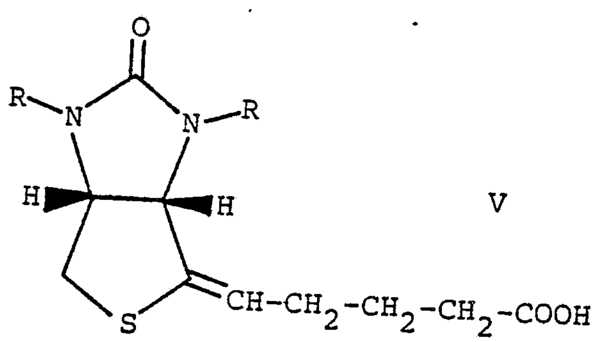

V

worin R die obige Bedeutung hat, in D-(+)-Biotin überführt.

**2.** Verfahren nach Anspruch 1 zur Herstellung einer Verbindung der Formel

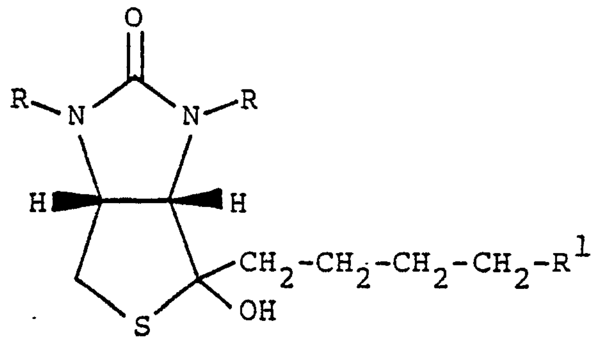

III

worin R und $R^1$ die in Anspruch 1 angegebene Bedeutung haben, dadurch gekennzeichnet, dass man das Thiolacton der Formel

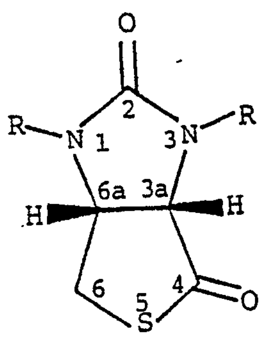

I

worin R die obige Bedeutung hat, mit einer Grignard-Verbindung der Formel

$$X-Mg-CH_2-CH_2-CH_2-CH_2-R^1 \qquad II$$

worin X Halogen darstellt und $R^1$ die obige Bedeutung hat, umsetzt.

**3.** Verfahren nach Anspruch 1 zur Herstellung einer Verbindung der Formel

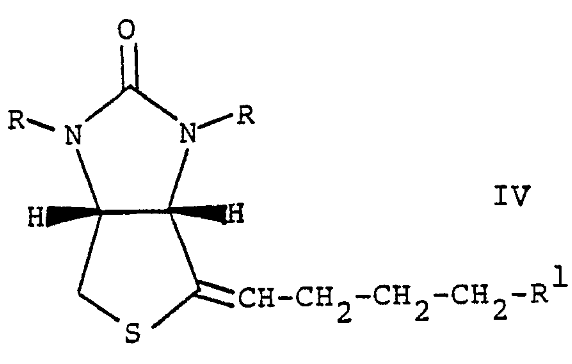

IV

worin R und $R^1$ die in Anspruch 1 angegebene Bedeutung haben, dadurch gekennzeichnet, dass man das Thiolacton der Formel

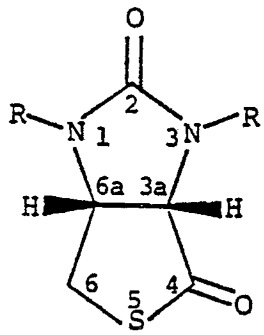

I

worin R die obige Bedeutung hat, mit einer Grignard-Verbindung der Formel

$$X{-}Mg{-}CH_2{-}CH_2{-}CH_2{-}CH_2{-}R^1 \qquad II$$

worin X Halogen darstellt und $R^1$ die obige Bedeutung hat, umsetzt und die so erhaltene Verbindung der Formel

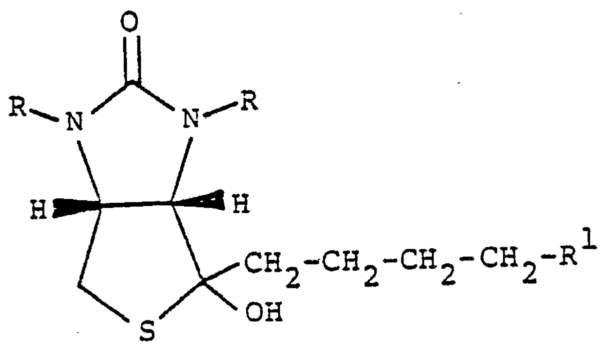

III

worin R und $R^1$ die obige Bedeutung haben, dehydratisiert.

**4.** Verfahren nach Anspruch 1 zur Herstellung einer Verbindung der Formel

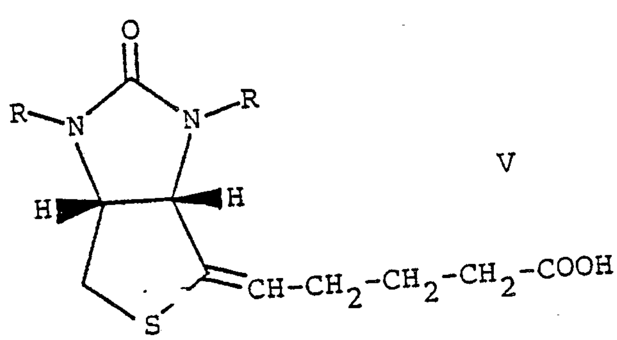

V

worin R die in Anspruch 1 angegebene Bedeutung hat, dadurch gekennzeichnet, dass man das Thiolacton der Formel

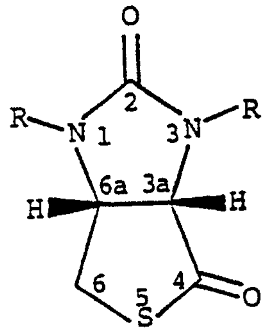

I

worin R die obige Bedeutung hat, mit einer Grignard-Verbindung der Formel

$$X\text{-}Mg\text{-}CH_2\text{-}CH_2\text{-}CH_2\text{-}CH_2\text{-}R^1 \qquad II$$

worin X Halogen darstellt und $R^1$ die obige Bedeutung hat, umsetzt und die so erhaltene Verbindung der Formel

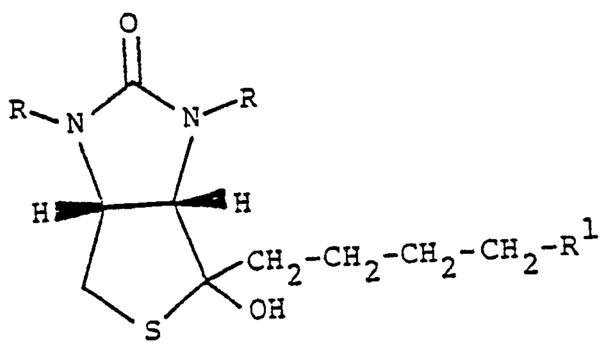

III

worin R und $R^1$ die obige Bedeutung haben, dehydratisiert und in der so erhaltenen Verbindung der Formel

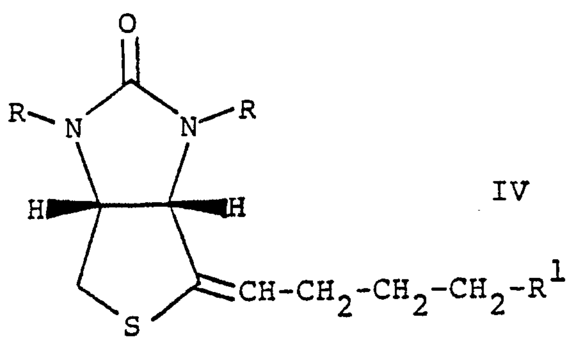

IV

worin R und $R^1$ die obige Bedeutung haben, durch Abspaltung von cis-1,3,5-Cyclohexantriol, die Carboxylgruppe der Seitenkette freisetzt.

**5.** Verfahren nach Anspruch 1 oder 4 , dadurch gekennzeichnet, dass man die Abspaltung des Cis-1,3,5-Cyclohexantriols mit einer Kombination aus wässriger Mineralsäure und einer katalytischen Menge P-Toluolsulfonsäure, gefolgt von einer Behandlung mit wässriger Alkalihydroxidlösung durchführt.

6. Verfahren nach Anspruch 5, dadurch gekennzeichnet, dass man als Mineralsäure Schwefelsäure, Chlorwasserstoffsäure oder Bromwasserstoffsäure verwendet.

7. Die Verbindung der Formel

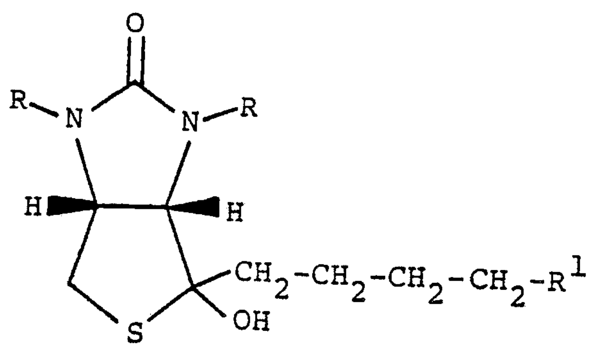

III

worin R die Benzylgruppe darstellt und $R^1$ den Rest der Formel

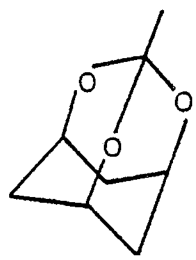

bedeutet.

8. Die Verbindung der Formel

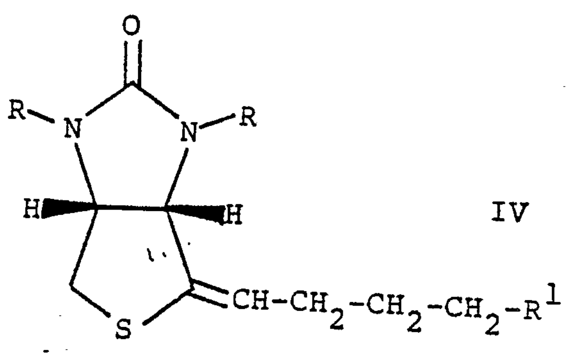

IV

worin R die Benzylgruppe darstellt und $R^1$ den Rest der Formel

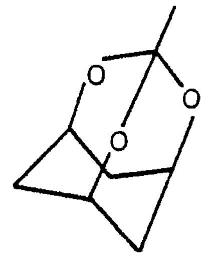

bedeutet.

Patentanspruch für folgenden Vertragsstaat: AT

**1.** Verfahren zur Herstellung von D-(+)-Biotin, dadurch gekennzeichnet, dass man das Thiolacton der Formel

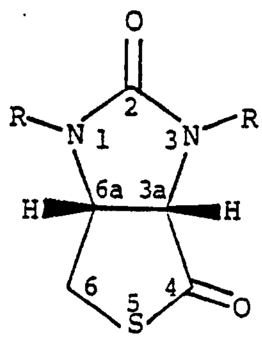

I

worin R die Benzylgruppe darstellt, mit einer Grignard-Verbindung der Formel

$$X\text{-}Mg\text{-}CH_2\text{-}CH_2\text{-}CH_2\text{-}CH_2\text{-}R^1 \quad \text{II}$$

worin X Halogen darstellt und $R^1$ den Rest der Formel

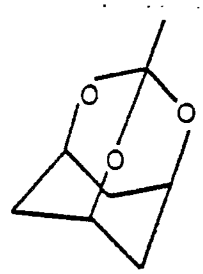

bedeutet,
umsetzt, dass man, die so erhaltene Verbindung der Formel

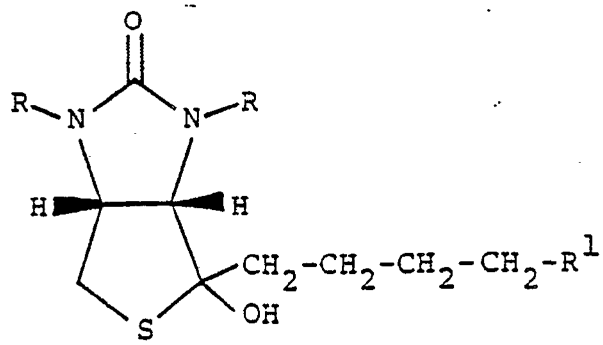

III

worin R und $R^1$ die obige Bedeutung haben, dehydratisiert, dass man, in der so erhaltenen Verbindung der Formel

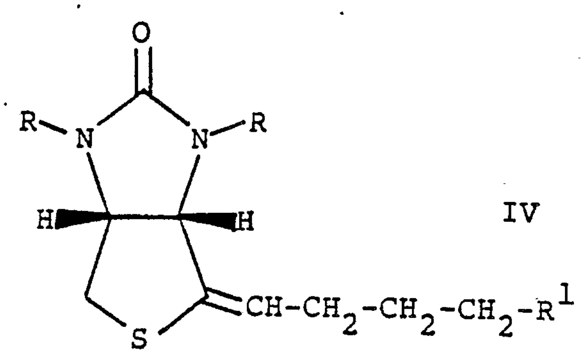

IV

worin R und $R^1$ die obige Bedeutung haben, durch Abspaltung von cis-1,3,5-Cyclohexantriol, die Garboxylgruppe der Seitenkette freisetzt, und die so erhaltene Verbindung der Formel

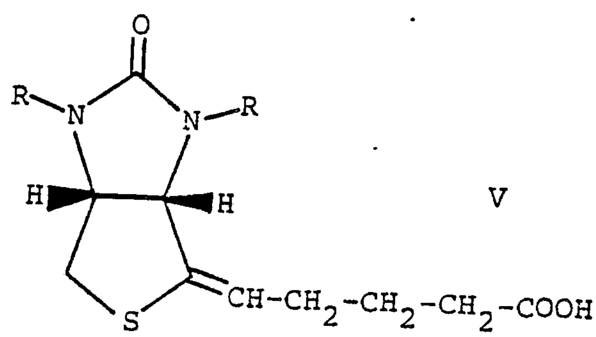

V

worin R die obige Bedeutung hat, in D-(+)-Biotin überführt.

**2.** Verfahren nach Anspruch 1 zur Herstellung einer Verbindung der Formel

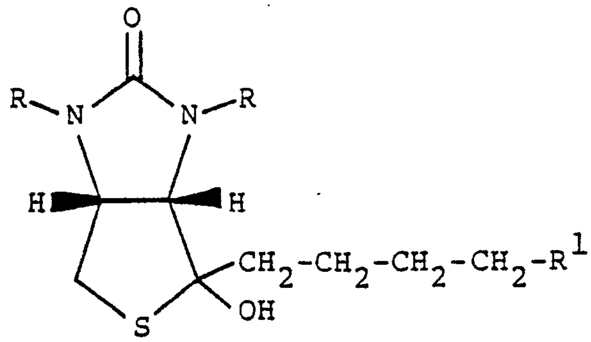

III

worin R und $R^1$ die in Anspruch 1 angegebene Bedeutung haben, dadurch gekennzeichnet, dass man das Thiolacton der Formel

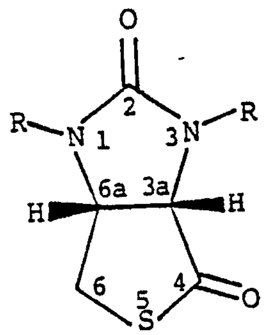

I

worin R die obige Bedeutung hat, mit einer Grignard-Verbindung der Formel

$$X\text{-}Mg\text{-}CH_2\text{-}CH_2\text{-}CH_2\text{-}CH_2\text{-}R^1 \qquad II$$

worin X Halogen darstellt und $R^1$ die obige Bedeutung hat, umsetzt.

3. Verfahren nach Anspruch 1 Herstellung einer Verbindung der Formel

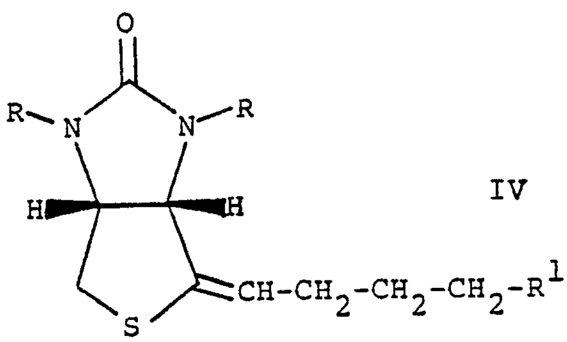

IV

worin R und $R^1$ die in Anspruch 1 angegebene Bedeutung haben, dadurch gekennzeichnet, dass man das Thiolacton der Formel

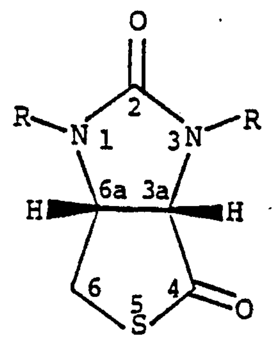

I

worin R die obige Bedeutung hat, mit einer Grignard-Verbindung der Formel

$$X\text{-}Mg\text{-}CH_2\text{-}CH_2\text{-}CH_2\text{-}CH_2\text{-}R^1 \qquad II$$

worin X Halogen darstellt und $R^1$ die obige Bedeutung hat, umsetzt und die so erhaltene Verbindung der Formel

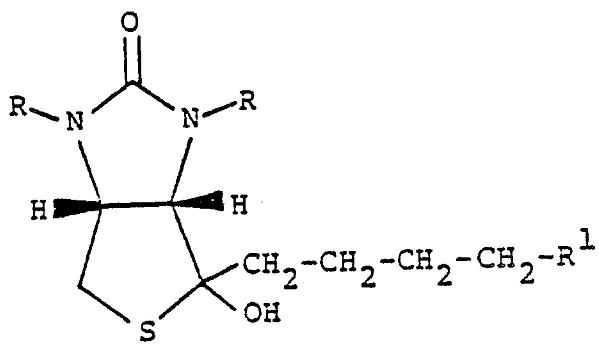

III

worin R und $R^1$ die obige Bedeutung haben, dehydratisiert.

**4.** Verfahren nach Anspruch 1 zur Herstellung einer Verbindung der Formel

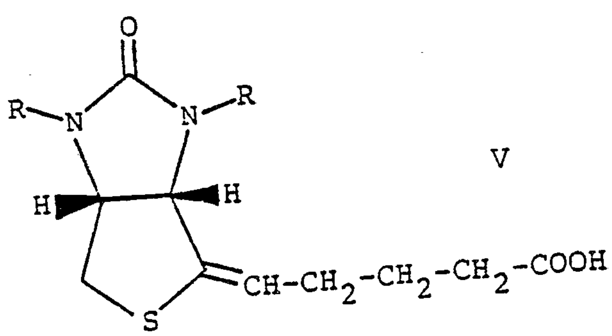

V

worin R die in Anspruch 1 angegebene Bedeutung hat, dadurch gekennzeichnet, dass man das Thiolacton der Formel

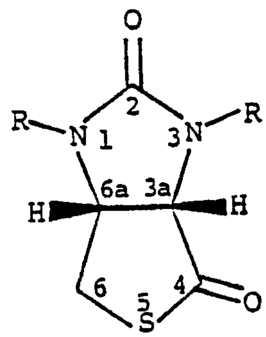

I

worin R die obige Bedeutung hat, mit einer Grignard-Verbindung der Formel

$$X\text{-}Mg\text{-}CH_2\text{-}CH_2\text{-}CH_2\text{-}CH_2\text{-}R^1 \qquad II$$

worin X Halogen darstellt und $R^1$ die obige Bedeutung hat, umsetzt und die so erhaltene Verbindung der Formel

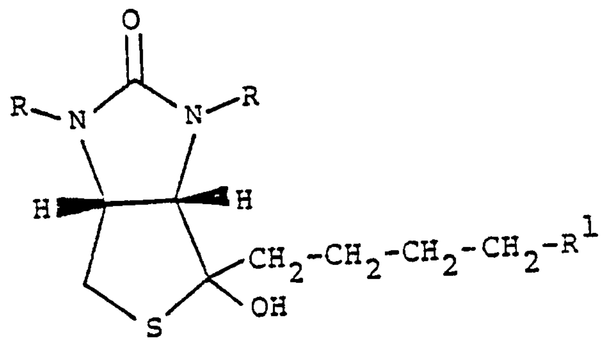

III

worin R und $R^1$ die obige Bedeutung haben, dehydratisiert und in der so erhaltenen Verbindung der Formel

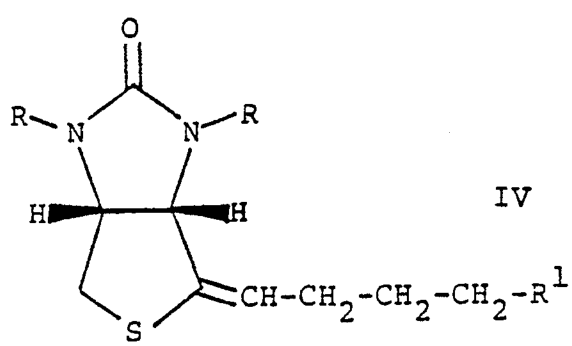

IV

worin R und $R^1$ die obige Bedeutung haben, durch Abspaltung von cis-1,3,5-Cyclohexantriol, die Carboxylgruppe der Seitenkette freisetzt.

**5.** Verfahren nach Anspruch 1 oder 4, dadurch gekennzeichnet, dass man die Abspaltung des cis-1,3,5-Cyclohexantriols mit einer Kombination aus wässriger Mineralsäure und einer katalytischen Menge p-Toluolsulfonsäure, gefolgt von einer Behandlung mit wässriger Alkalihydroxidlösung durchführt.

6. Verfahren nach Anspruch 5, dadurch gekennzeichnet, dass man als Mineralsäure Schwefelsäure, Chlorwasserstoffsäure oder Bromwasserstoffsäure verwendet.

## Claims

1. A process for the manufacture of D-(+)-biotin, characterized by reacting the thiolactone of the formula

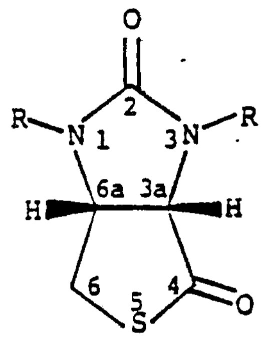

I

wherein R represents the benzyl group, with a Grignard compound of the formula

$$X\text{-}Mg\text{-}CH_2\text{-}CH_2\text{-}CH_2\text{-}CH_2\text{-}R^1 \qquad II$$

wherein X represents halogen and $R^1$ signifies the residue of the formula

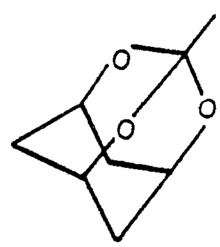

dehydrating the thus-obtained compound of the formula

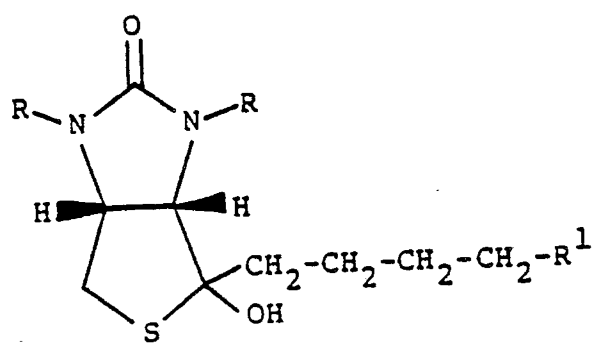

III

wherein R and $R^1$ have the above significance, liberating the carboxyl group in the side chain in the thus-obtained compound of the formula

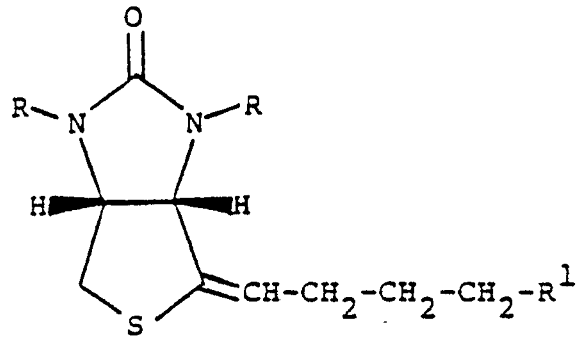

IV

wherein R and R' have the above significance, by the cleavage of cis-1,3,5-cyclohexanetriol and converting the thus-obtained compound of the formula

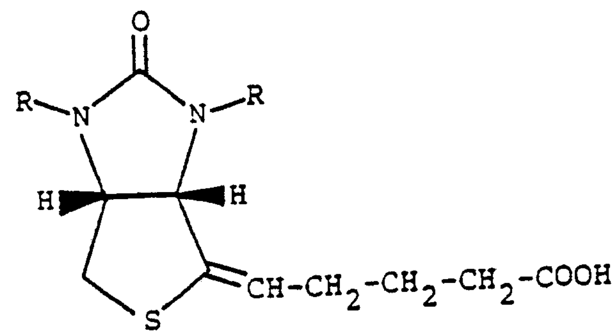

V

wherein R has the above significance, into D-(+)-biotin.

**2.** A process according to claim 1 for the manufacture of a compound of the formula

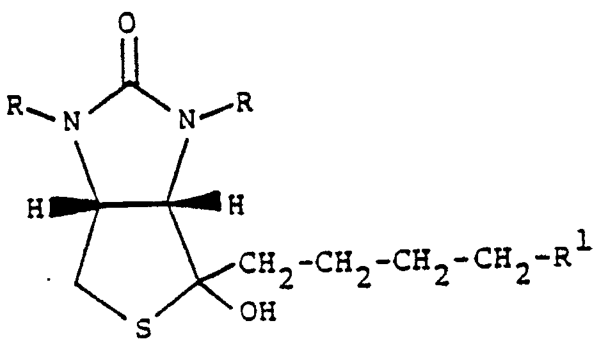

III

wherein R and $R^1$ have the significance given in claim 1, characterized by reacting the thiolactone of the formula

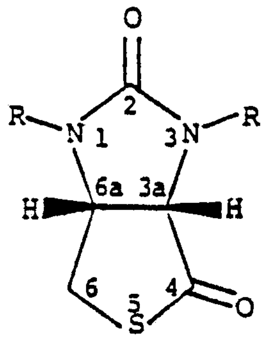

I

wherein R has the above significance, with a Grignard compound of the formula

$$X\text{-}Mg\text{-}CH_2\text{-}CH_2\text{-}CH_2\text{-}CH_2\text{-}R^1 \qquad II$$

wherein X represents halogen and $R^1$ has the above significance.

**3.** A process according to claim 1 for the manufacture of a compound of the formula

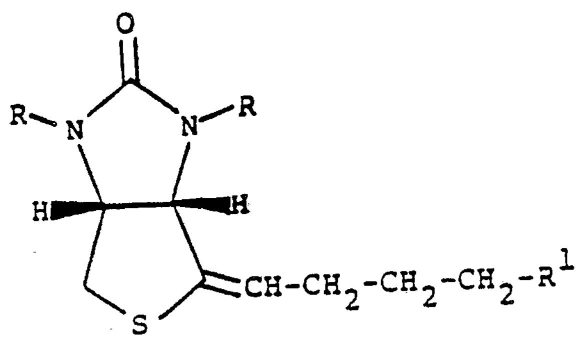

IV

wherein R and $R^1$ have the significance given in claim 1, characterized by reacting the thiolactone of the formula

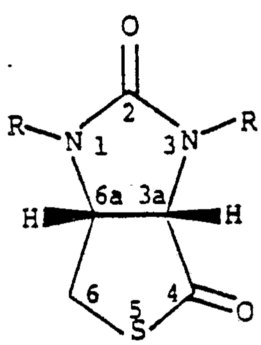

I

wherein R has the above significance, with a Grignard compound of the formula

$$X\text{-}Mg\text{-}CH_2\text{-}CH_2\text{-}CH_2\text{-}CH_2\text{-}R^1 \qquad II$$

wherein X represents halogen and $R^1$ has the above significance, and dehydrating the thus-obtained compound of the formula

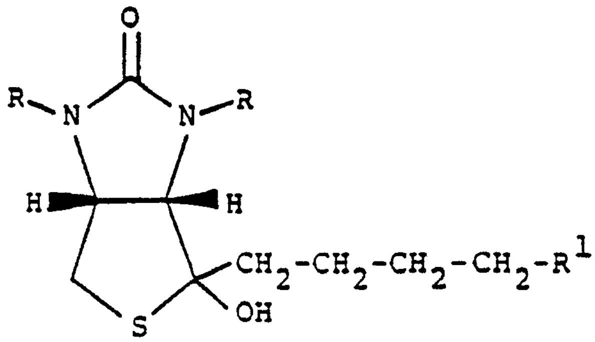

III

wherein R and $R^1$ have the above significance.

**4.** A process according to claim 1 for the manufacture of a compound of the formula

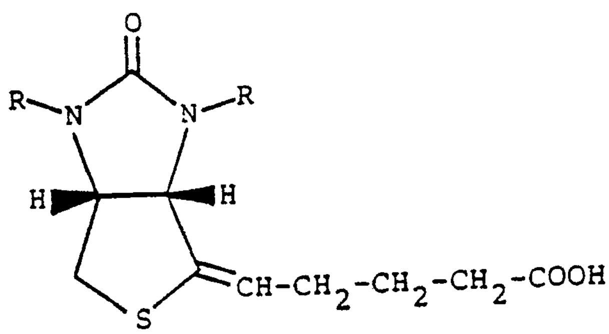

V

wherein R has the significance given in claim 1, characterized by reacting the thiolactone of the formula

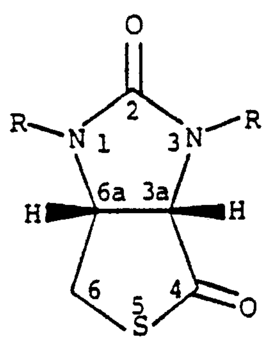

I

wherein R has the above significance, with a Grignard compound of the formula

$$X\text{-}Mg\text{-}CH_2\text{-}CH_2\text{-}CH_2\text{-}CH_2\text{-}R^1 \qquad II$$

wherein X represents halogen and $R^1$ has the above significance, and dehydrating the thus-obtained compound of the formula

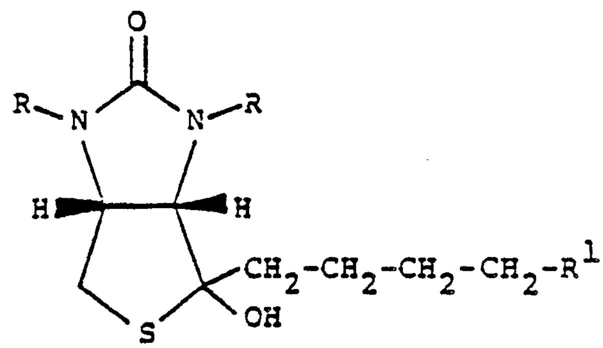

III

wherein R and $R^1$ have the above significance, and liberating the carboxyl group in the side chain in the thus-obtained compound of the formula

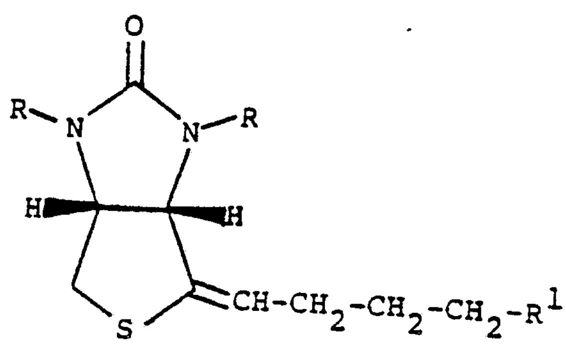

IV

wherein R and $R^1$ have the above significance, by the cleavage of cis-1,3,5-cyclohexanetriol.

5. A process according to claim 1 or 4, characterized in that the cleavage of the cis-1,3,5-cyclohexanetriol is carried out with a combination of aqueous mineral acid and a catalytic amount of p-toluenesulphonic acid, followed by a treatment with aqueous alkali hydroxide solution.

6. A process according to claim 5, characterized in that sulphuric acid, hydrochloric acid or hydrobromic acid is used as the mineral acid.

7. The compound of the formula

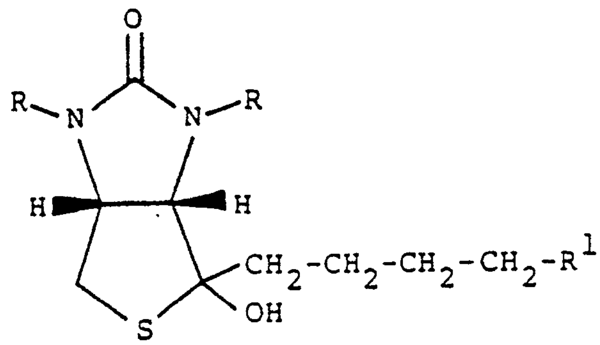

III

wherein R represents the benzyl group and $R^1$ signifies the residue of the formula

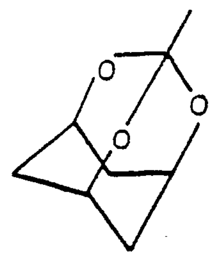

8. The compound of the formula

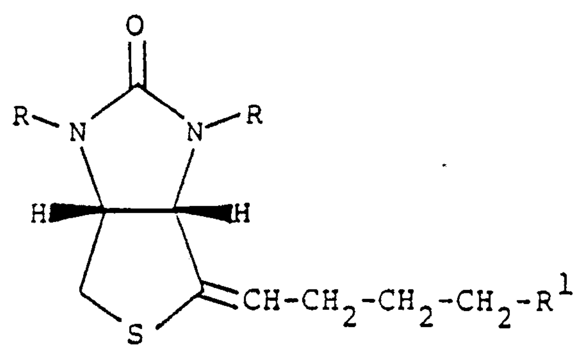

IV

wherein R represents the benzyl group and $R^1$ signifies the residue of the formula

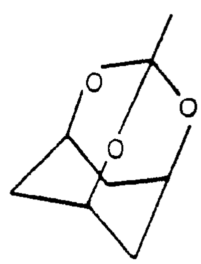

Claim for the following Contracting State: AT

**1.** A process for the manufacture of D-(+)-biotin, characterized by reacting the thiolactone of the formula

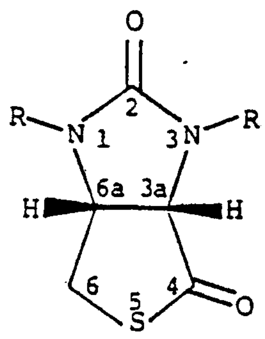

I

wherein R represents the benzyl group, with a Grignard compound of the formula

$$X-Mg-CH_2-CH_2-CH_2-CH_2-R^1 \qquad II$$

wherein X represents halogen and $R^1$ signifies the residue of the formula

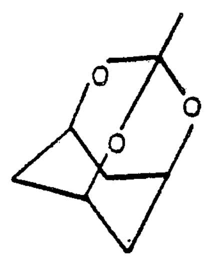

dehydrating the thus-obtained compound of the formula

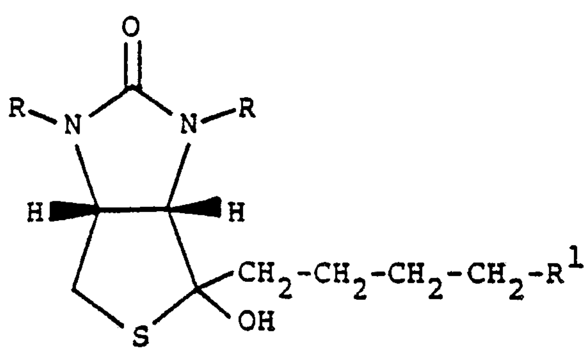

III

wherein R and $R^1$ have the above significance, liberating the carboxyl group in the side chain in the thus-obtained compound of the formula

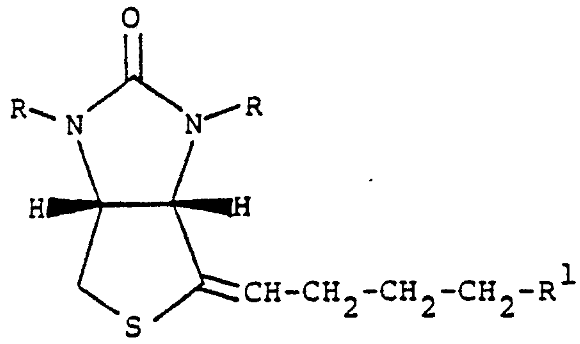

IV

wherein R and R¹ have the above significance, by the cleavage of cis-1,3,5-cyclohexanetriol and converting the thus-obtained compound of the formula

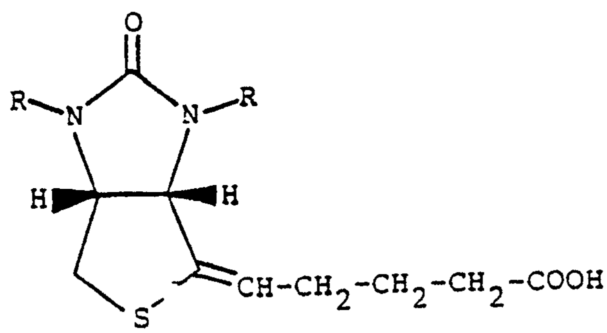

V

wherein R has the above significance, into D-(+)-biotin.

2. A process according to claim 1 for the manufacture of a compound of the formula

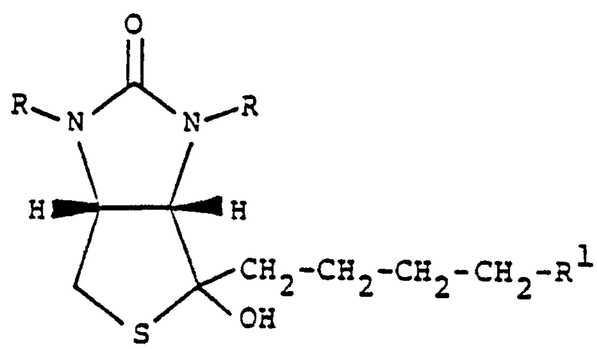

III

wherein R and $R^1$ have the significance given in claim 1, characterized by reacting the thiolactone of the formula

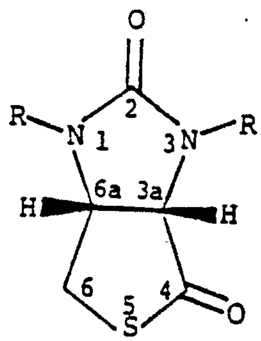

I

wherein R has the above significance, with a Grignard compound of the formula

$$X\text{-}Mg\text{-}CH_2\text{-}CH_2\text{-}CH_2\text{-}CH_2\text{-}R^1 \qquad II$$

wherein X represents halogen and $R^1$ has the above significance.

3. A process according to claim 1 for the manufacture of a compound of the formula

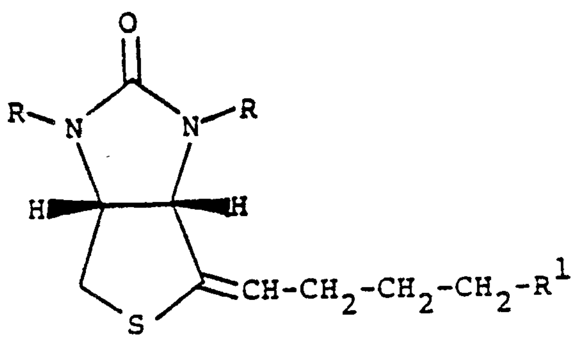

IV

wherein R and $R^1$ have the significance given in claim 1, characterized by reacting the thiolactone of the formula

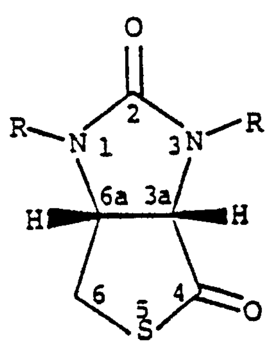

I

wherein R has the above significance. with a Grignard compound of the formula

$$X\text{-}Mg\text{-}CH_2\text{-}CH_2\text{-}CH_2\text{-}CH_2\text{-}R^1 \qquad II$$

wherein X represents halogen and $R^1$ has the above significance, and dehydrating the thus-obtained compound of the formula

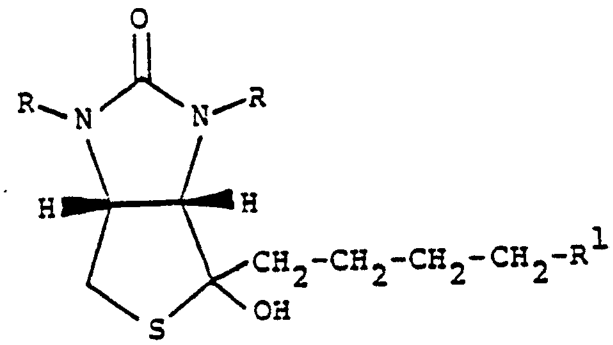

III

wherein R and $R^1$ have the above significance.

4. A process according to claim 1 for the manufacture of a compound of the formula

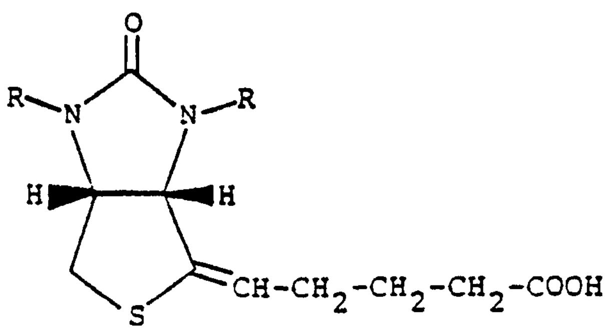

V

wherein R has the significance given in claim 1, characterized by reacting the thiolactone of the formula

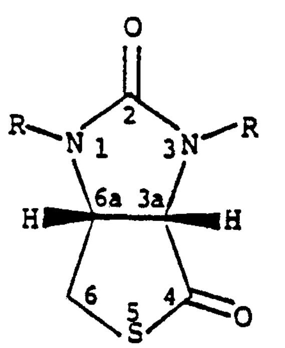

I

wherein R has the above significance, with a Grignard compound of the formula

$$X\text{-}Mg\text{-}CH_2\text{-}CH_2\text{-}CH_2\text{-}CH_2\text{-}R^1 \qquad II$$

wherein X represents halogen and $R^1$ has the above significance, and dehydrating the thus-obtained compound of the formula

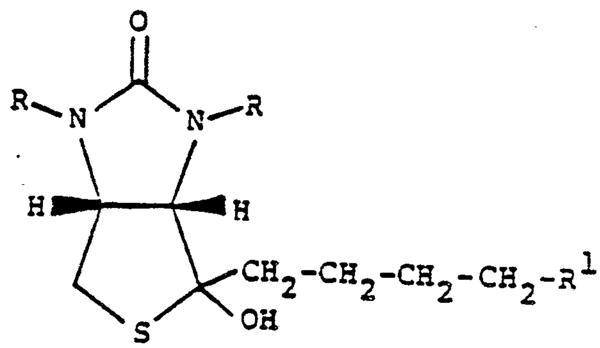

III

wherein R and $R^1$ have the above significance, and liberating the carboxyl group in the side chain in the thus-obtained compound of the formula

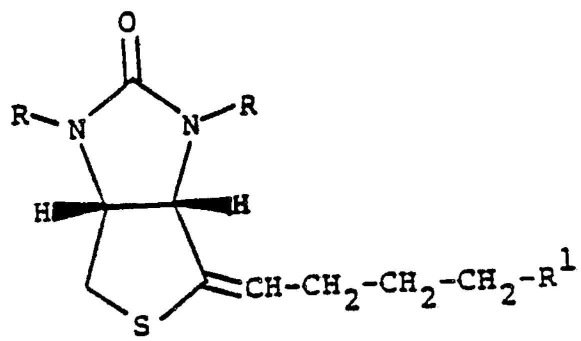

IV

wherein R and $R^1$ have the above significance. by the cleavage of cis-1,3,5-cyclohexanetriol.

5. A process according to claim 1 or 4, characterized in that the cleavage of the cis-1,3,5-cyclohexanetriol is carried out with a combination of aqueous mineral acid and a catalytic amount of p-toluenesulphonic acid, followed by a treatment with aqueous alkali hydroxide solution.

6. A process according to claim 5, characterized in that sulphuric acid, hydrochloric acid or hydrobromic acid is used as the mineral acid.

## Revendications

1. Procédé de préparation de la D-(+)-biotine, caractérisé en ce que l'on fait réagir la thiolactone de formule

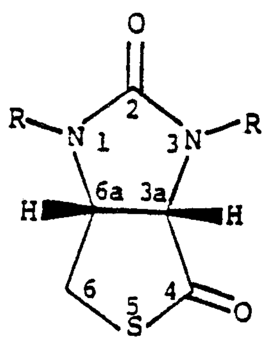

I

dans laquelle R représente le groupe benzyle, avec un réactif de Grignard de formule

$$X\text{-}Mg\text{-}CH_2\text{-}CH_2\text{-}CH_2\text{-}CH_2\text{-}R^1 \qquad II$$

dans laquelle X représente un halogène et $R^1$ le groupe de formule

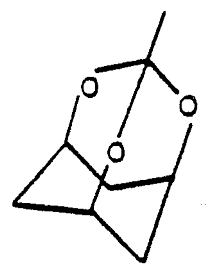

ce qui donne le composé de formule

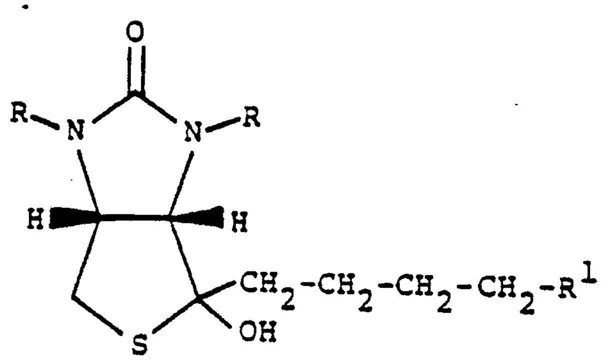

III

dans laquelle R et $R^1$ ont les significations indiquées ci-dessus, qu'on soumet à déshydratation, ce qui donne le composé de formule

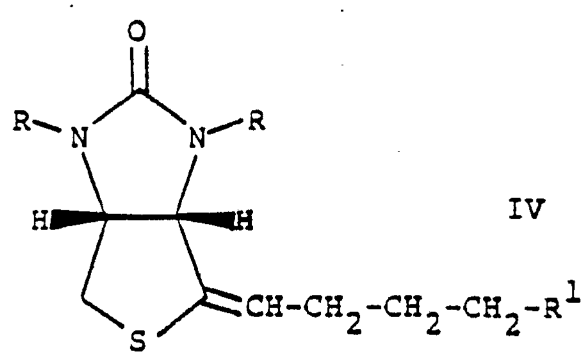

IV

dans laquelle R et $R^1$ ont les significations indiquées ci-dessus, d'où, par scission du cis-1,3,5-cyclohexane-triol, on libère le groupe carboxyle de la chaîne latérale, ce qui donne le composé de formule

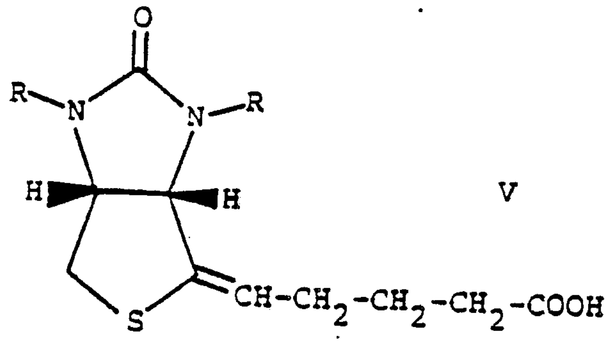

V

dans laquelle R a les significations indiquées ci-dessus, qu'on convertit en la D-(+)-biotine.

**2.** procédé selon la revendication 1, pour préparer un composé de formule

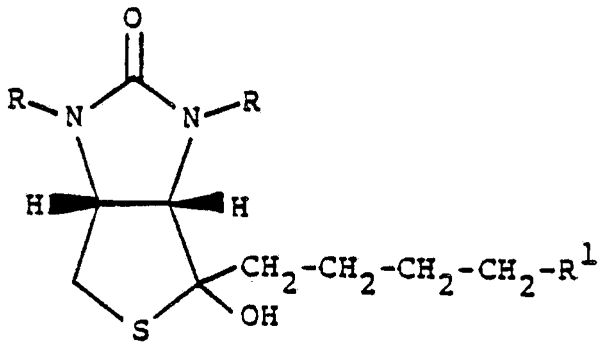

III

dans laquelle R et $R^1$ ont les significations indiquées dans la revendication 1, caractérisé en ce que l'on fait réagir la thiolactone de formule

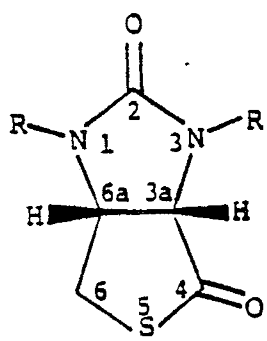

I

dans laquelle R a les significations indiquées ci-dessus, avec un réactif de Grignard de formule

$$X\text{-}Mg\text{-}CH_2\text{-}CH_2\text{-}CH_2\text{-}CH_2\text{-}R^1 \qquad II$$

dans laquelle X représente un halogène et $R^1$ a les significations indiquées ci-dessus

**3.** procédé selon la revendication 1, pour préparer un composé de formule

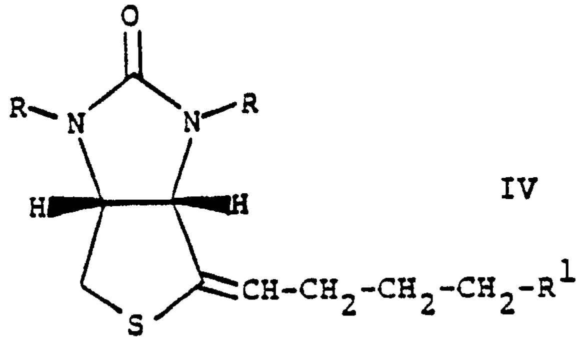

IV

dans laquelle R et $R^1$ ont les significations indiquées dans la revendication 1, caractérisé en ce que l'on fait réagir la thiolactone de formule

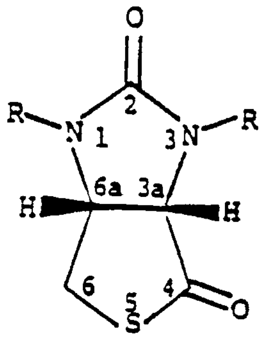

I

dans laquelle R a les significations indiquées ci-dessus, avec un réactif de Grignard de formule

$$X\text{-}Mg\text{-}CH_2\text{-}CH_2\text{-}CH_2\text{-}CH_2\text{-}R^1 \qquad II$$

dans laquelle X représente un halogène et $R^1$ a les significations indiquées ci-dessus, ce qui donne un composé de formule

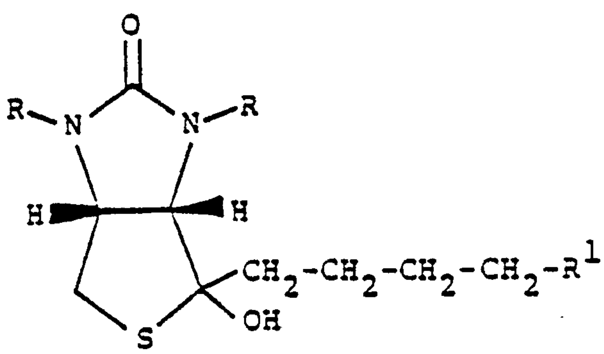

III

dans laquelle R et $R^1$ ont les significations indiquées ci-dessus, qu'on soumet à déshydration.

4. Procédé selon la revendication 1, pour préparer un composé de formule

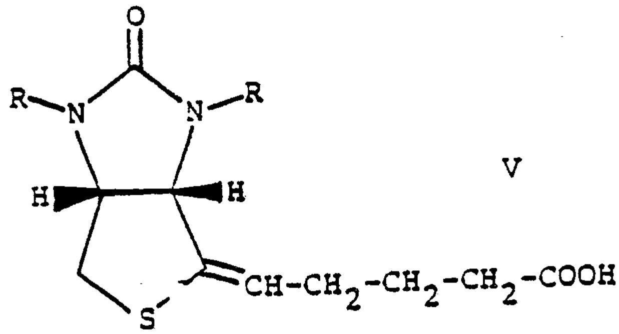

V

dans laquelle R a les significations indiquées dans la revendication 1, caractérisé en ce que l'on fait réagir la thiolactone de formule

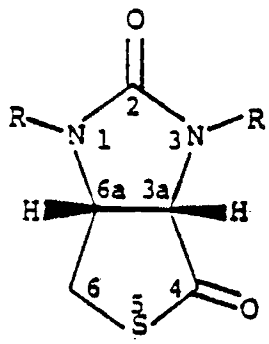

I

dans laquelle R a les significations indiquées ci-dessus, avec un réactif de Grignard de formule

$$X\text{-}Mg\text{-}CH_2\text{-}CH_2\text{-}CH_2\text{-}CH_2\text{-}R^1 \qquad II$$

dans laquelle X représente un halogène et $R^1$ a les significations indiquées ci-dessus, ce qui donne un composé de formule

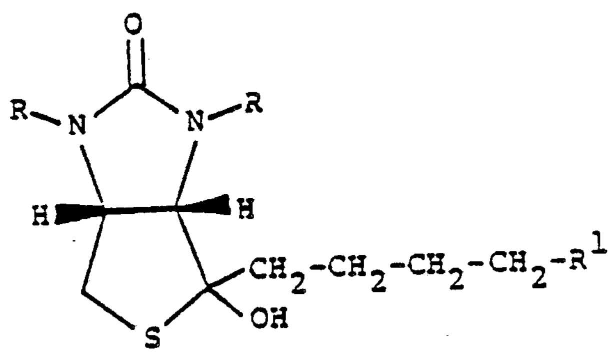

III

dans laquelle R et $R^1$ ont les significations indiquées ci-dessus, qu'on soumet à déshydration, ce qui donne le composé de formule

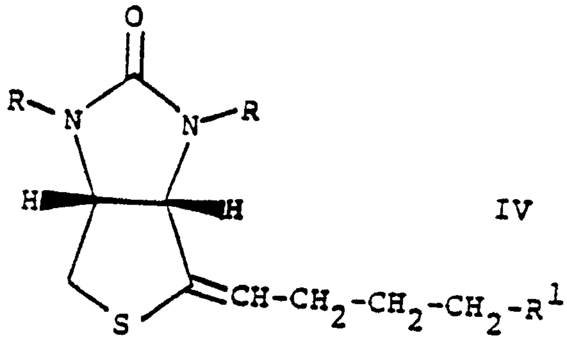

IV

dans laquelle R et $R^1$ ont les significations indiquées ci-dessus, d'où, par scission du cis-1,3,5-cyclohexane-triol, on libère le groupe carboxyle de la chaîne latérale.

**5.** procédé selon la revendication 1 ou 4, caractérisé en ce que l'on scinde le cis-1,3,5-cyclohexane-triol à l'aide d'une combinaison d'un acide minéral aqueux et d'une quantité catalytique d'acide p-toluène-sulfonique, en faisant suivre d'un traitement par une solution aqueuse d'hydroxyde alcalin.

**6.** Procédé selon la revendication 5, caractérisé en ce que l'acide minéral utilisé est l'acide sulfurique, l'acide chlorhydrique ou l'acide bromhydrique.

**7.** Le composé de formule

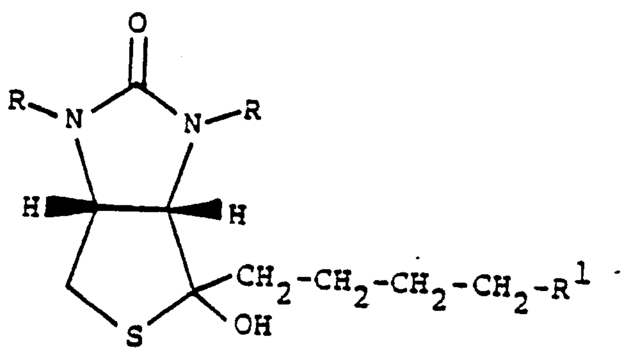

III

dans laquelle R représente le groupe benzyle et $R^1$ le groupe de formule

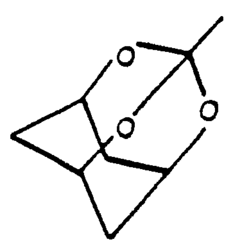

**8.** Le composé de formule

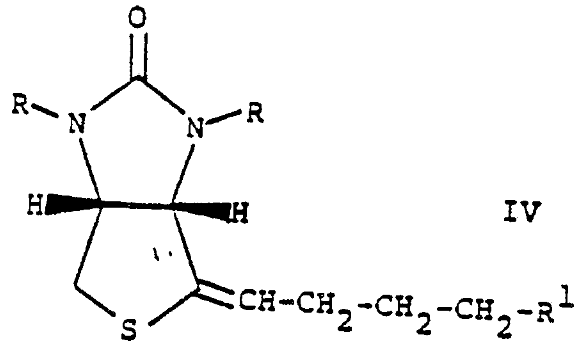

IV

dans laquelle R représente le groupe benzyle et $R^1$ le groupe de formule

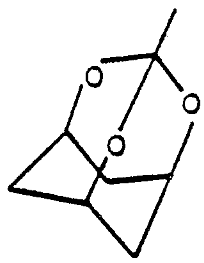

Revendications pour l'Etat contractant suivant: AT

1. Procédé de préparation de la D-(+)-biotine, caractérisé en ce que l'on fait réagir la thiolactone de formule

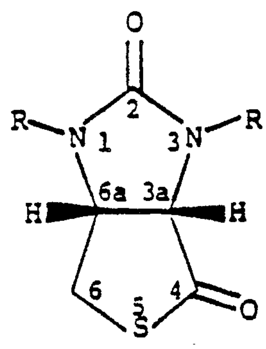

I

dans laquelle R représente le groupe benzyle, avec un réactif de Grignard de formule

$$X\text{-}Mg\text{-}CH_2\text{-}CH_2\text{-}CH_2\text{-}CH_2\text{-}R^1 \qquad II$$

dans laquelle X représente un halogène et $R^1$ le groupe de formule

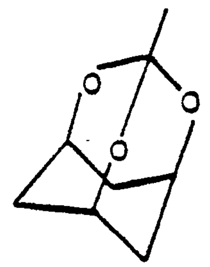

ce qui donne le composé de formule

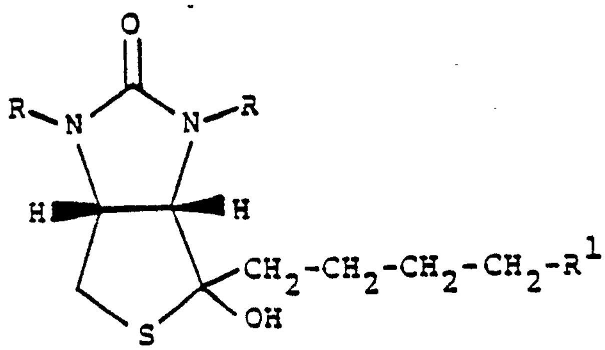

III

dans laquelle R et $R^1$ ont les significations indiquées ci-dessus, qu'on soumet à déshydratation, ce qui donne le composé de formule

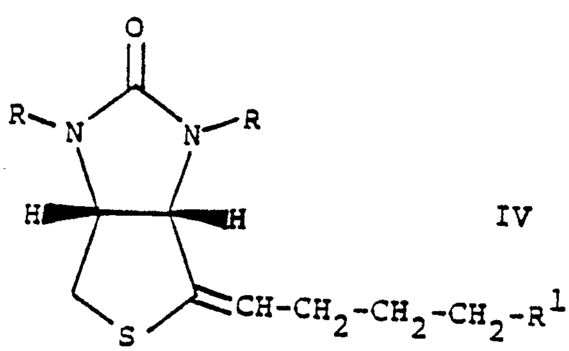

IV

dans laquelle R et $R^1$ ont les significations indiquées ci-dessus, d'où, par scission du cis-1,3,5-cyclohexane-triol, on libère le groupe carboxyle de la chaîne latérale, ce qui donne le composé de formule

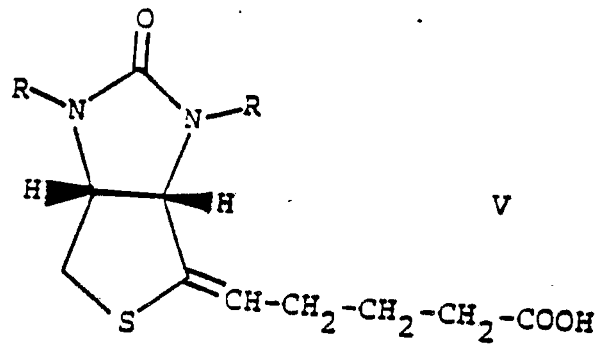

V

dans laquelle R a les significations indiquées ci-dessus, qu'on convertit en la D-(+)-biotine.

2. Procédé selon la revendication 1, pour préparer un composé de formule

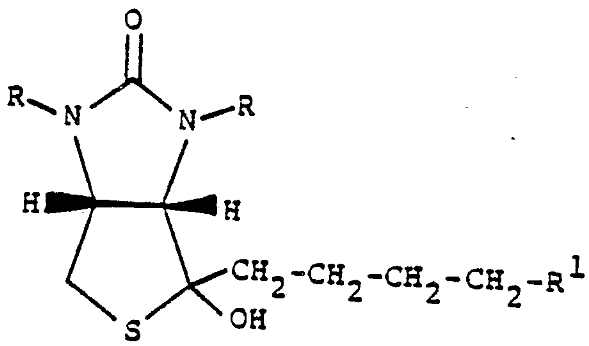

III

dans laquelle R et $R^1$ ont les significations indiquées dans la revendication 1, caractérisé en ce que l'on fait réagir la thiolactone de formule

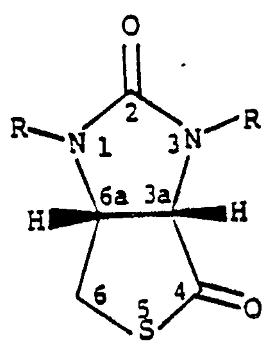

I

dans laquelle R a les significations indiquées ci-dessus, avec un réactif de Grignard de formule

$$X\text{-}Mg\text{-}CH_2\text{-}CH_2\text{-}CH_2\text{-}CH_2\text{-}R^1 \qquad II$$

dans laquelle X représente un halogène et $R^1$ a les significations indiquées ci-dessus

3. procédé selon la revendication 1, pour préparer un composé de formule

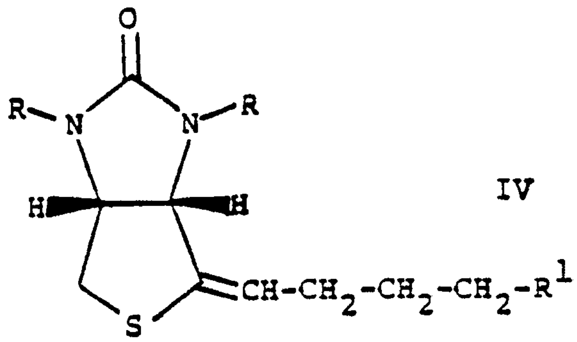

IV

dans laquelle R et $R^1$ ont les significations indiquées dans la revendication 1, caractérisé en ce que l'on fait réagir la thiolactone de formule

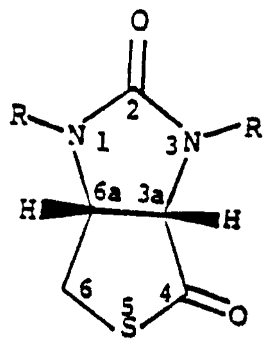

I

dans laquelle R a les significations indiquées ci-dessus, avec un réactif de Grignard de formule

$$X\text{-}Mg\text{-}CH_2\text{-}CH_2\text{-}CH_2\text{-}CH_2\text{-}R^1 \qquad II$$

dans laquelle X représente un halogène et $R^1$ a les significations indiquées ci-dessus, ce qui donne le composé de formule

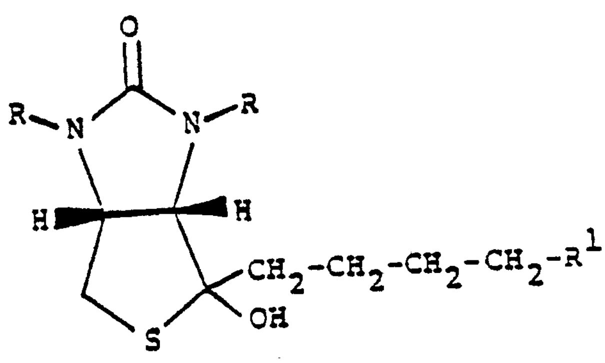

III

dans laquelle R et $R^1$ ont les significations indiquées ci-dessus,qu'on soumet à déshydration.

**4.** Procédé selon la revendication 1, pour préparer un composé de formule

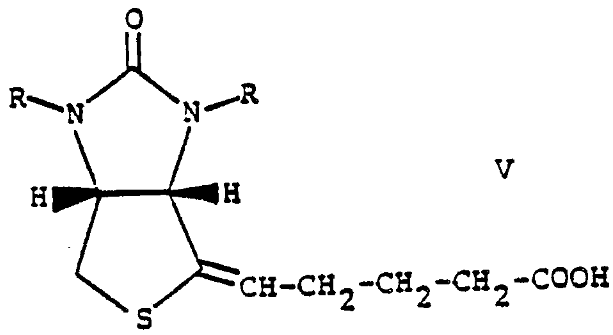

V

dans laquelle R a les significations indiquées dans la revendication 1, caractérisé en ce que l'on fait réagir la thiolactone de formule

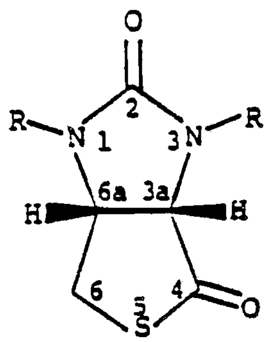

I

dans laquelle R a les significations indiquées ci-dessus, avec un réactif de Grignard de formule

$$X\text{-}Mg\text{-}CH_2\text{-}CH_2\text{-}CH_2\text{-}CH_2\text{-}R^1 \qquad II$$

dans laquelle X représente un halogène et $R^1$ a les significations indiquées ci-dessus, ce qui donne le composé de formule

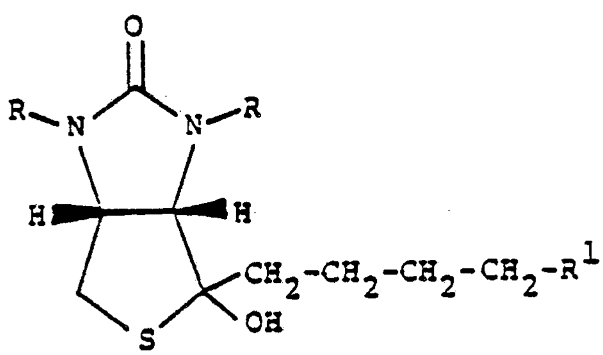

III

dans laquelle R et $R^1$ ont les significations indiquées ci-dessus, qu'on soumet à déshydratation, ce qui donne le composé de formule

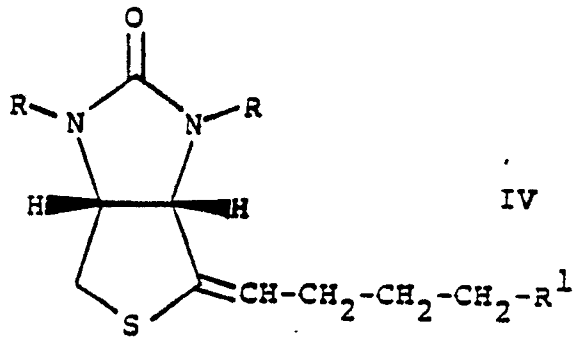

IV

dans laquelle R et $R^1$ ont les significations indiquées ci-dessus, d'où, par scission du cis-1,3,5-cyclohexane-triol, on libère le groupe carboxyle de la chaîne latérale.

5. Procédé selon la revendication 1 ou 4, caractérisé en ce que l'on scinde le cis-1,3,5-cyclohexane-triol à l'aide d'une combinaison d'un acide minéral aqueux et d'une quantité catalytique d'acide p-toluène-sulfonique, en faisant suivre d'un traitement par une solution aqueuse d'hydroxyde alcalin.

6. Procédé selon la revendication 5, caractérisé en ce que l'acide minérale utilisé est l'acide sulfurique, l'acide chlorhydrique ou l'acide bromhydrique.